# EUROPÄISCHE PATENTSCHRIFT

(11) **EP 1 663 919 B1**
(45) Veröffentlichungstag und Bekanntmachung des Hinweises auf die Patenterteilung: **02.12.2009**
(21) Anmeldenummer: 04765000.7
(22) Anmeldetag: 09.09.2004
(51) Int. Cl.: C07C 13/66, C07C 25/22, C07C 205/06, C07D 333/08, C09K 11/06, H05B 33/14

(54) **VERBINDUNGEN AUF BASIS VON FLUORANTHEN UND IHRE VERWENDUNG**
COMPOUNDS BASED ON FLUORANTHENE AND USE THEREOF
COMPOSES A BASE DE FLUORANTHENE ET LEUR UTILISATION

(30) Priorität: 11.09.2003 DE 10342340
(43) Veröffentlichungstag der Anmeldung: 07.06.2006
(73) Patentinhaber: BASF SE, 67056 Ludwigshafen (DE)
(72) Erfinder: SCHWALM, Reinhold, 67157 Wachenheim (DE); HEISCHKEL, Yvonne, 68199 Mannheim (DE); FECHTENKÖTTER, Andreas, 67063 Ludwigshafen (DE); RÖSCH, Joachim, 67063 Ludwigshafen (DE); DÖTZ, Florian, 69120 Heidelberg (DE)
(74) Vertreter: Isenbruck, Günter
(86) Internationale Anmeldenummer: PCT/EP2004/010065
(87) Internationale Veröffentlichungsnummer: WO 2005/026088

(56) Entgegenhaltungen:
- EP-A- 1 138 745
- EP-A- 1 345 477
- WO-A-03/105538
- DE-A1- 2 166 862
- US-A- 3 190 887
- US-A- 5 281 489
- US-A- 5 965 746
- US-A1- 2002 022 151
- DATABASE CA [Online] CHEMICAL ABSTRACTS SERVICE, COLUMBUS, OHIO, US; LAALI, KENNETH K. ET AL: "Stable ion and electrophilic chemistry of fluoranthene-PAHs" XP002318634 gefunden im STN Database accession no. 2002:289507 & JOURNAL OF THE CHEMICAL SOCIETY, PERKIN TRANSACTIONS 2 , (3), 621-629 CODEN: JCSPGI; ISSN: 1472-779X, 2002,
- DATABASE CA [Online] CHEMICAL ABSTRACTS SERVICE, COLUMBUS, OHIO, US; CURTZE, JUERGEN ET AL: "The synthesis of fluoranthenes and related aromatic polycycles by means of pyridinium salts" XP002318635 gefunden im STN Database accession no. 1979:575059 & CHEMISCHE BERICHTE , 112(6), 2197-208 CODEN: CHBEAM; ISSN: 0009-2940, 1979,
- DATABASE CA [Online] CHEMICAL ABSTRACTS SERVICE, COLUMBUS, OHIO, US; ZHUKHOVITSKII, V. B. ET AL: "Haloacyl derivatives of polycyclic hydrocarbons. V. Condensations using haloacylfluoranthenes" XP002318636 gefunden im STN Database accession no. 1974:403879 & VOPROSY KHIMII I KHIMICHESKOI TEKHNOLOGII , 30, 27-31 CODEN: VKKCAJ; ISSN: 0321-4095, 1973,
- DATABASE CA [Online] CHEMICAL ABSTRACTS SERVICE, COLUMBUS, OHIO, US; SHENBOR, M. I.: "Fluoranthenyl-4-acetic acid and its derivatives" XP002318637 gefunden im STN Database accession no. 1970:435111 & ZHURNAL ORGANICHESKOI KHIMII , 6(5), 1079-81 CODEN: ZORKAE; ISSN: 0514-7492, 1970,
- DATABASE CA [Online] CHEMICAL ABSTRACTS SERVICE, COLUMBUS, OHIO, US; DAVIES, ALUN ET AL: "Nitration of indeno[1,2,3-cd]fluoranthene" XP002318638 gefunden im STN Database accession no. 1969:19315 & JOURNAL OF THE CHEMICAL SOCIETY [SECTION] B: PHYSICAL ORGANIC , (11), 1337-9 CODEN: JCSPAC; ISSN: 0045-6470, 1968,
- DATABASE CA [Online] CHEMICAL ABSTRACTS SERVICE, COLUMBUS, OHIO, US; BUU-HOI, N. P. ET AL: "Friedel-Crafts acylation reactions of polycyclic aromatic hydrocarbons. V. Acetylation of fluoranthene" XP002318639 gefunden im STN Database accession no. 1968:496322 & BULLETIN DE LA SOCIETE CHIMIQUE DE FRANCE , (3), 981-4 CODEN: BSCFAS; ISSN: 0037-8968, 1968,
- E. CLAR ET AL: "The location of double bonds in fluoranthene perylene and 1, 1'-dinaphthyl by NMR" TETRAHEDRON, Bd. 25, 1969, Seiten 5639-5648, XP002318632
- K. PRAEFCKE ET AL: "Eine ungewöhnliche Bildung des Fluoranthen-Ringsystems" TETRAHEDRON LETTERS, Nr. 21, 1976, Seiten 1787-1788, XP002318633
- DATABASE BEILSTEIN BEILSTEIN INSTITUT ZUR FOERDERUNG DER CHEMISCHEN WISSENSCHAFTEN, FRANKFURT AM MAIN, DE; XP002318640 Database accession no. BRN 1396647 UND 1396671 & N.P. BUU-HOI ET AL: J. CHEM. SOC., 1964, Seiten 3920-3924,
- DATABASE BEILSTEIN BEILSTEIN INSTITUT ZUR FOERDERUNG DER CHEMISCHEN WISSENSCHAFTEN, FRANKFURT AM MAIN, DE; XP002318641 Database accession no. BRN 259939 & N.P. BUU-HOI ET AL: J. CHEM. SOC., 1958, Seiten 2946-1948,
- DATABASE BEILSTEIN BEILSTEIN INSTITUT ZUR FOERDERUNG DER CHEMISCHEN WISSENSCHAFTEN, FRANKFURT AM MAIN, DE; XP002318642 Database accession no. BRN 1911745 UND 1911746 & CAMPBELL ET AL: CHEM. IND., 1970, Seite 1114,
- DATABASE BEILSTEIN BEILSTEIN INSTITUT ZUR FOERDERUNG DER CHEMISCHEN WISSENSCHAFTEN, FRANKFURT AM MAIN, DE; XP002318643 Database accession no. BRN: 3870240,3318645 & BUU-HOI ET AL: CHEM.BER., Bd. 77, 1944, Seiten 121-125,

## Beschreibung

Die vorliegende Erfindung betrifft Fluoranthenderivate, Verfahren zu ihrer Herstellung sowie die Verwendung von Fluoranthenderivaten als Emittermolekül in organischen lichtemittierenden Dioden (OLEDs), eine lichtemittierende Schicht enthaltend die erfindungsgemäßen Fluoranthenderivate als Emittermoleküle, eine OLED enthaltend die erfindungsgemäße lichtemittierende Schicht sowie Vorrichtungen enthaltend die erfindungsgemäße OLED.

In organischen lichtemittierenden Dioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen.

Es wurden zahlreiche Materialien vorgeschlagen, die bei Anregung durch elektrischen Strom Licht emittieren.

Eine Übersicht über organische lichtemittierende Dioden ist z. B. in M. T. Bernius et al., Adv. Mat. 2000, 12, 1737 offenbart. Die Anforderungen an die verwendeten Verbindungen sind dabei hoch und es gelingt mit den bekannten Materialien üblicherweise nicht, alle gestellten Anforderungen zu erfüllen.

In US 5,281,489 sind OLEDs offenbart, die als fluoreszierende Materialien unter anderem 3,4-Benzofluoranthen oder monomeres unsubstituiertes Fluoranthen enthalten können. Monomeres unsubstituiertes Fluoranthen kann jedoch unter den in den OLEDs herrschenden Anwendungsbedingungen migrieren. Die Schicht aus monomerem unsubstituierten Fluoranthen ist nicht stabil, woraus eine geringe Lebensdauer der Dioden resultiert.

Der Einsatz spezieller Fluoranthenderivate ist in US 2002/0022151 A1 und EP-A 1 138 745 offenbart.

EP-A 1 138 745 betrifft eine OLED, die rötliches Licht emittiert. Diese OLED enthält eine organische Schicht, die eine Verbindung mit einem Fluoranthengerüst aufweist, wobei das Fluoranthengerüst mit mindestens einer Aminogruppe oder einer Alkenylgruppe substituiert ist. Bevorzugt sind gemäß der Beschreibung solche Fluoranthenderivate, die mindestens 5, bevorzugt mindestens 6 kondensierte Ringe aufweisen. Diese Verbindungen emittieren Licht längerer Wellenlänge, so dass gelbes bis rötliches Licht emittiert werden kann. Die in EP-A 1 138 745 offenbarten Fluoranthenderivaten tragen bevorzugt eine Aminogruppe zur Erhöhung der Lebensdauer der Fluoranthenderivate.

US 2002/0022151 A1 betrifft ebenfalls OLEDs, die als lichtemittierendes Material spezielle Fluoranthenverbindungen enthalten. Diese Fluoranthenverbindungen weisen mindestens eine Diarylanunogruppe auf.

Aufgabe der vorliegenden Erfindung ist es, Verbindungen bereit zu stellen, die als Emittermoleküle in OLEDs geeignet sind, die eine lange Lebensdauer aufweisen und die in OLEDs hoch effizient sind.

Diese Aufgabe wird durch die Verwendung von Fluoranthenderivaten der allgemeinen Formel (I) 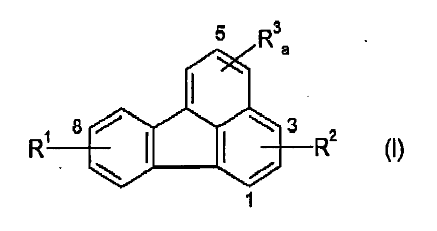 gelöst, worin a = 0 ist und R¹ und R² und a unabhängig voneinander die folgenden Bedeutungen aufweisen:
- R¹, R²: eine mit linearen, verzweigten oder zyklischen C₁- bis C₂₀-Alkylgruppen, die wiederum mit Halogen-, Nitro-, Ether- oder Carboxygruppen substituiert oder unsubstituiert sein können und/oder worin ein oder mehrere Kohlenstoffatome der Alkylgruppen durch Si, P, O oder S ersetzt sein können, mit Alkoxygruppen oder mit C₆ - bis C₁₄-Arylgruppen substituierte oder unsubstituierte C₆-Arylgruppe,
und wobei die Fluoranthenderivate Substituenten aufweisen, die über eine C-C-Einfachbindung mit dem Fluoranthengerüst verknüpft sind, als Emittermolekül in organischen Licht-emittierenden Dioden (OLEDs).

Die erfindungsgemäßen Fluoranthenderivate weisen Substituenten auf, die über eine C-C-Einfachbindung mit dem Fluoranthengerüst verknüpft sind. Die erfindungsgemäßen Fluoranthenderivate sind überraschenderweise hinreichend stabil, um in einer lichtemittierenden Schicht in OLEDs mit einer langen Lebensdauer eingesetzt zu werden. Des Weiteren zeichnen sich die erfindungs gemäßen Verbindungen durch eine sehr hohe Stabilität gegenüber Photooxidation bei der Verwendung in OLEDs aus.

Besonders bevorzugt sind R¹ und R² C₆-Arylgruppen, die mit Halogen, bevorzugt Cl oder F, oder Nitrogruppen substituiert sind. Ganz besonders bevorzugt tragen diese Arylgruppen eine bis drei Halogen- oder Nitrogruppen, ganz besonders bevorzugt eine oder zwei Halogen- oder Nitrogruppen. Insbesondere bevorzugt ist eine C₆-Arylgruppe, die mit einer oder zwei Halogen- oder Nitrogruppen substituiert ist. Die Substituenten der C₆-Arylgruppe sind - bei einem Substituenten - bevorzugt in 4-Position der Arylgruppe, - bei zwei Substituenten - bevorzugt in 3- und 5-Position. Weiterhin bevorzugt ist eine C₆-Arylgruppe, die unsubstituiert ist (also eine Phenylgruppe).

Die Reste R¹ und R² können unabhängig voneinander aus den vorstehend genannten Resten ausgewählt sein, mit der Maßgabe, dass mindestens einer der Reste R¹ oder R² nicht Wasserstoff ist, das heißt, unsubstituiertes Fluoranthen ist von den erfindungsgemäßen Verbindungen nicht umfasst.

Ganz besonders bevorzugt sind R¹ und R² unabhängig voneinander eine mit linearen oder verzweigten unsubstituierten C₁- bis C₈-Alkylgruppen, Halogen- oder Nitrogruppen substituierte oder unsubstituierte C₆-Arylgruppe.

In einer weiteren ganz besonders bevorzugten Ausführungsform sind R¹ und R² unabhängig voneinander eine mit linearen oder verzweigten unsubstituierten C₁- bis C₈-Alkylgruppen, Halogen- oder Nitrogruppen substituierte oder unsubstituierte Phenylgruppe, die besonders bevorzugt keinen, einen oder zwei Substituenten trägt.

a in der allgemeinen Formel I ist 0, das heißt das Fluoranthenderivat der allgemeinen Formel I enthält zwei Substituenten, nämlich R¹ und R². Diese Substituenten sind in einer ganz besonders bevorzugten Ausführungsform in der 3- und der 8-Position des Fluoranthengerüsts angeordnet Dabei ist die 3-Position mit dem Rest R² substituiert und die 8-Position mit dem Rest R¹. Ganz besonders bevorzugt sind weder R¹ noch R² Wasserstoff.

In einer ganz besonders bevorzugten Ausführungsform umfasst die vorliegende Erfindung Fluorantenderivate, die in der 3- und in der 8-Position eine mit einem oder zwei Substituenten substituierte Phenylgruppe tragen.

Insbesondere bevorzugt sind Fluoranthenderivate, die in der 3- und 8- Position eine substituierte Phenylgruppe tragen, wobei die Phenylgruppe bevorzugt einen oder zwei Substituenten trägt. Die Substituenten der Phenylgruppe sind bereits vorstehend erwähnt. Ganz besonders bevorzugt sind als Substituenten Halogengruppen wie Fluor, Chlor oder Brom, bevorzugt Fluor, Nitrogruppen oder aromatische Gruppen wie Phenyl- oder Biphenylreste. Ist die substituierte Phenylgruppe mit einem Substituenten substituiert, so erfolgt die Substitution ganz besonders bevorzugt in der 4'-Position des Phenylrests, d. h. in p-Stellung zum Fluoranthenrest. Ist die substituierte Phenylgruppe mit zwei Substituenten substituiert, so erfolgt die Substitution ganz besonders bevorzugt in 3'- und 5'- Position.

Es wurde überraschenderweise gefunden, dass Fluoranthenderivate, die in 3- und 8-Position eine substituierte Phenylgruppe tragen, Licht in der blauen Region des sichtbaren elektromagnetischen Spektrums emittieren.

Zur Herstellung von Displays, die die Farben des gesamten sichtbaren Spektrums umfassen, ist es erforderlich, OLEDs bereitzustellen, die Licht in der roten Region des sichtbaren elektromagnetischen Spektrums Licht emittieren, OLEDs, die in der grünen Region des sichtbaren elektromagnetischen Spektrums Licht emittieren und OLEDs, die in der blauen Region des sichtbaren elektromagnetischen Spektrums Licht emittieren. Es hat sich herausgestellt, dass insbesondere die Bereitstellung von effizienten OLEDs, die Licht in der blauen Region des sichtbaren elektromagnetischen Spektrums emittieren, problematisch ist.

Die erfindungsgemäß verwendeten Fluoranthenderivate, die in 3- und 8- Position eine substituierte Phenylgruppe tragen, sind zur Herstellung von OLEDs, die Licht in der blauen Region des sichtbaren elektromagnetischen Spektrums emittieren, geeignet.

Die erfindungsgemäß verwendeten Fluoranthenderivate der allgemeinen Formel I können durch Umsetzung von an den Positionen, an denen in den gewünschten Fluoranthenderivaten der allgemeinen Formel I die Reste R¹ und R² mit dem Fluoranthengerüst verknüpft sind, halogenierten, insbesondere bromierten, Fluoranthenderivaten mit den den gewünschten Resten R¹ und R² entsprechenden Boronsäuren oder Boronsäureestern unter Pd(0)-Katalyse in Gegenwart einer Base hergestellt werden (Suzuki-Kupplung). Eine Substitution von Fluoranthenderivaten durch Knüpfung einer Kohlenstoff-Kohlenstoff-Bindung mittels Suzuki-Kupplung ist aus dem Stand der Technik bisher nicht bekannt.

Anstelle der den gewünschten Resten R¹ und R² entsprechenden Boronsäuren oder Boronsäureester können auch andere borhaltige Verbindungen, die die gewünschten Reste R¹ und R² tragen, in der Umsetzung mit den halogenierten Fluoranthenderivaten eingesetzt werden. Bei den borhaltigen Verbindungen handelt es sich um Verbindungen der allgemeinen Formeln R¹-B(O-[C(R⁴)₂]ₙ)-O, R²-B(O-[C(R⁴)₂]ₙ)-O und gegebenenfalls R³-B(O-[C(R⁴)₂]ₙ)-O, worin
R¹ und R² die für R¹ und R² vorstehend genannten Bedeutungen aufweisen,
R⁴ gleich oder verschieden ist und ausgewählt aus Wasserstoff oder C₁-C₂₀-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Decyl, n-Dodecyl oder n-Octadecyl; bevorzugt C₁-C₁₂-Alkyl wie Methyl, Ethyl, n-Propyl, iso-Propyl, n-Buyl, iso-Butyl, sec.-Butyl, tert-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec-Butyl und tert.-Butyl, ganz besonders bevorzugt Methyl; und
n eine ganze Zahl von 2 bis 10, vorzugsweise 2 bis 5 ist.

Es ist ebenfalls möglich, die Suzuki-Kupplung durch Umsetzung von an den Positionen, an denen in den gewünschten Fluoranthenderivaten der allgemeinen Formel I die Reste R¹ und R² mit dem Fluoranthengerüst verknüpft sind, mit Boronsäure- oder Boronestergruppen oder Gruppen der Formel -B{O-[C(R⁴)₂]ₙ)-O substituierten Fluoranthendenvaten mit den den gewünschten Resten R¹ und R² entsprechenden Halogeniden unter Pd(O)-Katalyse in Gegenwart einer Base durchzuführen. R⁴ weist die vorstehend genannten Bedeutungen auf.

Bevorzugt erfolgt die Suzuki-Kupplung in der erstgenannten Ausführungsform.

Die den gewünschten Resten R¹ und R² oder den Fluoranthenderivaten entsprechenden Boronsäuren und Boronsäureester können nach dem Stand der Technik bekannten Verfahren hergestellt werden. Beispielsweise ist die Herstellung der Boronsäuren und Boronsäureester durch Umsetzung von Grignard- oder Lithium-Reagenzien mit Boranen, Diboranen oder Boraten möglich. Die Herstellung der anderen geeigneten borhaltigen Verbindungen erfolgt nach dem Fachmann bekannten Verfahren.

Die Herstellung der halogenierten, bevorzugt bromierten, Fluoranthenderivate ist dem Fachmann bekannt. Beispielsweise kann eine Bromierung durch Umsetzung von Fluoranthen in einem Lösungsmittel, z. B. Chloroform, mit elementarem Brom erfolgen. Ein Verfahren zur Bromierung von Fluoranthenderivaten ist z. B. in DE-A 32 09 426 offenbart. Die den Resten R¹ und R² entsprechenden Halogenide sind käuflich zu erwerben bzw. nach dem Fachmann bekannten Verfahren erhältlich.

Als Pd(0)-Katalysatoren sind alle üblichen Pd(0)-Katalysatoren geeignet. Beispielsweise können Tris(dibenzylidenaceton)-dipalladium(0) oder Tetrakis(triphenylphosphin)-palladium(0) eingesetzt werden. Die Katalysatoren werden im allgemeinen in einer Menge von 0,001 bis 15 mol-%, bevorzugt 0,01 bis 10 mol-%, besonders bevorzugt 0,1 bis 5 mol-%, bezogen auf die Fluoranthenverbindung, eingesetzt.

In der Suzuki-Kupplung können alle üblicherweise in der Suzuki-Kupplung eingesetzten Basen eingesetzt werden. Bevorzugt werden Alkalicarbonate wie Natrium- oder Kaliumcarbonat eingesetzt. Die Base wird im Allgemeinen im molaren Überschuß von 20- bis 200-fach, bevorzugt 20- bis 100-fach, besonders bevorzugt 20- bis 80-fach, bezogen auf die Fluoranthenverbindungen, eingesetzt.

Die den gewünschten Resten R¹ und R² entsprechende Komponente (Boronsäure, der entsprechende Boronsäureester oder die anderen geeigneten borhaltigen Verbindungen bzw. die entsprechenden Halogenide) werden in einem Verhältnis zu dem halogenierten oder mit Boronsäure- oder Boronestergruppen oder Gruppen der Formel -B(O-[C(R⁴)₂]ₙ)-0 substituierten Fluoranthenderivat von 100 bis 400 mol-%, bevorzugt 100 bis 300 mol-%, besonders bevorzugt 100 bis 150 mol-% eingesetzt.

Die Umsetzung erfolgt im Allgemeinen bei einer Temperatur von 40 bis 140 °C, bevorzugt 60 bis 120 °C, besonders bevorzugt 80 bis 100. Der Druck beträgt im Allgemeinen Normaldruck.

Die Umsetzung wird im Allgemeinen unter Ausschluss von Sauerstoff durchgeführt. Üblicherweise erfolgt die Umsetzung in einem Lösungsmittel ausgewählt aus der Gruppe bestehend aus Benzol, Toluol, Tetrahydrofuran, 1,4-Dioxan und Petrolether.

In einer besonders bevorzugten Ausführungsform des erfindungsgemäßen Verfahrens wird ein halogeniertes Fluoranthen in Lösung unter Schutzgas vorgelegt und mit der Base, die bevorzugt gelöst, z. B. in einem Ethanol/Wasser-Gemisch, vorliegt, und einer Boronsäure versetzt. Im Anschluss daran wird unter Schutzgas der Pd(0)-Katalysator zugegeben. Es wird über einen Zeitraum von im Allgemeinen 2 bis 120 Stunden, bevorzugt 4 bis 72 Stunden, besonders bevorzugt 6 bis 48 Stunden bei den vorstehend genannten Temperaturen und Drucken gerührt. Im Anschluss daran wird das Reaktionsgemisch nach den dem Fachmann bekannten Verfahren aufgearbeitet.

Die Aufarbeitung kann z. B. in der Weise erfolgen, dass das Reaktionsgemisch auf einen Alkohol, bevorzugt Methanol, gegossen wird, anschließend filtriert wird und das Filtrat nach Trocknung, z. B. mit Molekularsieb oder MgSO₄, eingeengt und chromatographisch gereinigt wird.

Die erfindungsgemäß verwendeten Fluoranthenderivate können neben der Suzuki-Kupplung durch andere dem Fachmann bekannte Verfahren, insbesondere andere Kupplungsreaktionen, hergestellt werden.

In einer weiteren Ausführungsform der vorliegenden Erfindung werden die erfindungsgemäß verwendeten Fluoranthenderivate der Formel 1 durch Umsetzung von an den Positionen, an denen in den gewünschten Fluoranthenderivaten der allgemeinen Formel 1 die Reste R¹ und R² mit dem Fluoranthengerüst verknüpft sind, halogenierten, insbesondere bromierten, Fluoranthenderivaten mit den den gewünschten Resten R¹ und R² entsprechenden Brom-Verbindungen unter Ni(0)-Katalyse (Yamamoto-Kupplung) erhalten.

Eine Substitution von Fluoranthenderivaten durch Knüpfung einer Kohlenstoff-Kohlenstoff-Bindung mittels Suzuki-Kupplung oder Yamamoto-Kupplung ist aus dem Stand der Technik bisher nicht bekannt.

In einer bevorzugten Ausführungsform der Yamamoto-Kupplung wird unter Sauerstoffausschluss eine Lösung, bevorzugt eine DMF-Lösung, des Katalysators hergestellt aus einer Ni(0)-Verbindung, bevorzugt Ni(COD)₂, und Bipyridyl in äquimolaren Mengen eingesetzt. Zu dieser Lösung werden unter Sauerstoff-Ausschluss das halogenierte, bevorzugt bromierte, Fluoranthenderivat und die den gewünschten Resten R¹, R² und gegebenenfalls R³ entsprechenden Brom-Verbindungen in einem Lösungsmittel, bevorzugt Toluol, zugegeben.

Die Reaktionsbedingungen wie Temperatur, Druck, Lösungsmittel, Verhältnis der Fluoranthenkomponente zu der R¹ und R² entsprechenden Komponente, Ausschluss von Sauerstoff und Aufarbeitung bei der Herstellung der erfindungsgemäßen Fluoranthenderivate mittels Yamamoto-Kupplung entsprechen denen der Suzuki-Kupplung.

Als Ni(0)-Verbiudungen zur Herstellung des Katalysators sind alle üblichen Ni(0)-Verbindungen geeignet. Beispielsweise können Ni(C₂H₄)₃, Ni(1,5-Cyclooctadien)₂ ("Ni(COD)₂"), Ni(1,6-Cyclodecadien)₂ oder Ni(1,5,9-all-trans-Cyclododeratrien)₂ eingesetzt werden. Die Katalysatoren werden im Allgemeinen in einer Menge von 1 bis 100 mol-%, bevorzugt 5 bis 80 mol-%, besonders bevorzugt 10 bis 70 mol-%, bezogen auf die Fluoranthenverbindung, eingesetzt.

Durch die erfindungsgemäßen Verfahren ist es möglich, eine breite Palette von Fluoranthenderivaten bereitzustellen, die mittels C-C-Verkaüpfung substituiert sind. Somit können Fluoranthenderivate bereitgestellt werden, die mit der jeweils gewünschten Wellenlänge fluoreszieren.

Die erfindungsgemäß verwendeten Fluoranthendenvate zeichnen sich dadurch aus, dass sie geeignet sind, in organischen lichtemittierenden Dioden (OLEDs) elektromagnetische Strahlung im blauen Bereich des sichtbaren Spektrums zu emittieren.

Ein weiterer Gegenstand der vorliegenden Erfindung sind Fluoranthenderivate der allgemeinen Formel I, worin a = 0 ist und R¹ und R² unabhängig voneinander die folgenden Bedeutungen aufweisen:
- R¹, R²: eine mit linearen, verzweigten oder zyklischen C₁- bis C₂₀-Alkylgruppen, die wiederum mit Halogen-, Nitro-, Ether- oder Carboxygruppen substituiert oder unsubstituiert sein können und/oder worin ein oder mehrere Kohlenstoffatome der Alkylgruppen durch Si, P, 0 oder S ersetzt sein können, mit Alkoxygruppen oder mit C₆- bis C₁₄-Arylgruppen substituierte oder unsubstituierte C₆-Arylgruppe,
wobei das Fluoranthenderivat der allgemeinen Formel (I) in der 3-Position mit R² und in der 8-Position mit R¹ substituiert ist. Bevorzugte Fluoranthenderivate und deren Herstellungsverfahren sind bereits vorstehend genannt.

Organische lichtemittierenden Dioden sind grundsätzlich aus mehreren Schichten aufgebaut. Dabei sind verschiedene Schichtenfolgen möglich, z. B.:
- Anode/lochtransportierende Schicht/lichtemittierende Schicht/Kathode;
- Anode/lichtemittierende Schicht/elektronentransportierende Schicht/Kathode;
- Anode/lochtransportierende Schicht/lichtemittierende Schicht/elektronentransportierende Schicht/Kathode;

Die erfindungsgemäß verwendeten Fluoranthenderivate der allgemeinen Formel I werden bevorzugt in der lichtemittierenden Schicht als Emittermonoleküle eingesetzt. Ein weiterer Gegenstand der vorliegenden Erfindung ist daher eine lichtemittierende Schicht enthaltend ein oder mehrere Fluoranthenderivate der allgemeinen Formel I als Emittermoleküle. Bevorzugte Fluoranthenderivate sind bereits vorstehend genannt.

Die Fluoranthenderivate können selbst die lichtemittierende Schicht bilden. In der lichtemittierenden Schicht können, falls notwendig, zusätzlich zu den erfindungsgemäßen Fluoranthenderivaten übliche lichtemittierende Materialien, Dotierstoffe, lochtransportierende Substanzen und elektronentransportierende Substanzen eingesetzt werden. Die Fluoranthenderivate der allgemeinen Formel I gemäß der vorliegenden Anmeldung können jedoch auch als Dotierstoffe in der lichtemittierenden Schicht eingesetzt werden. Bevorzugt werden Fluoranthenderivate der allgemeinen Formel I in einer Konzentration von 1 bis 70 Gew.-%, bevorzugt von 1 bis 20 Gew.-% zu der lichtemittierenden Schicht zugegeben.

Die einzelnen vorstehend genannten Schichten des OLEDs können wiederum aus zwei oder mehreren Schichten aufgebaut sein. Beispielsweise kann die lochtransportierende Schicht aus einer Schicht aufgebaut sein, in die aus der Elektrode Löcher injiziert werden, nachstehend lochinjizierende Schicht genannt, und einer Schicht, die die Löcher von der lochinjizierenden Schicht weg in die lichtemittierende Schicht transportiert. Diese Schicht wird im Folgenden lochtransportierende Schicht genannt. Die elektronentransportierende Schicht kann ebenfalls aus mehreren Schichten bestehen, z. B. aus einer Schicht, worin Elektronen durch die Elektrode injiziert werden, im Folgenden elektroneninjizierende Schicht genannt, und einer Schicht, die aus der elektroneninjizierenden Schicht Elektronen erhält und in die lichtemittierende Schicht transportiert, im Folgenden elektronentransportierende Schicht genannt. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt.

Geeignete Materialien, die in der lichtemittierenden Schicht als Basismaterial in Kombination mit den erfindungsgemäßen Fluoranthenderivaten der allgemeinen Formel (I) eingesetzt werden, sind Anthracen, Naphthalin, Phenanthren, Pyren, Tetracen, Coronen, Krysen, Fluorescein, Perylen, Phthaloperylen, Naphthaloperylen, Perynon, Phthaloperynon, Naphthaloperynon, Diphenylbutadien, Tetraphenylbutadien, Cumarin, Oxadiazol, Aldazin, Bisbenzoxazolin, Bisstyryl, Pyrazin, Cyclopentadien, Metallkomplexe des Chinolins, Metallkomplexe von Aminochinolin, Metallkomplexe des Benzochinolins, Imine, Diphenylethylen, Vinylanthracen, Diaminocarbazol, Pyran, Thiopyran, Polymethin, Merocyanin, Chelate von oxinoiden Verbindungen mit Imidazolen, Chinacridon, Rubren, Stilbenderivate und fluoreszierende Pigmente.

Als lochtransportierendes Material wird im Allgemeinen eine Verbindung eingesetzt, die die Fähigkeit besitzt, die Löcher aus der Anode aufzunehmen Löcher zu transportieren und gleichzeitig dazu geeignet ist, Löcher in die lichtemittierende Schicht zu injizieren. Geeignete lochtransportierende Materialien sind beispielsweise Metallkomplexe des Phthalocyanins, des Naphthalocyanins, des Porphyrins, Pyrazolone, Tetrahydroimidazole, Hydrazone, Acylhydrazone, Polyarylalkane, Thiophene, tertiäre aromatische Amine wie Triphenylamine des Benzidintyps, Triphenylamine des Styrylamintyps, Triphenylamine des Diamintyps, Derivate dieser Verbindungen, Silanamine, insbesondere Silanamine, die Triphenylsilylgruppen tragen, und makromolekulare Verbindungen wie Polyvinylcarbazole, Polyvinylsilane, Polythiophen, Poly(p-phenylen) und leitfähige Makromoleküle. Besonders bevorzugte lochtransportierende Materialien sind z. B. in EP-A 1 138 745 und Chen et al. Macromol. Symp. 125, 9 bis 15 (1997) offenbart.

Als elektronentransportierende Materialien sind Verbindungen geeignet, die die Fähigkeit aufweisen Elektronen zu transportieren, und die selbst Elektronen in die lichtemittierende Schicht injizieren können. Geeignete elektronentransportierende Materialien sind beispielsweise Oxazole, Oxadiazole, Triazole, Imidazole, Imidazolone, Imidazolethione, Fluorenon, Anthrachinondimethan, Diphenochinon, Thiopyrandioxid, Oxazol, Oxadiazol, Triazol, Imidazol, Perylentetracarbonsäure, Fluorenylidenmethan, Distyrylarylene, Arylene, Cumarine und Derivate der genannten Verbindungen sowie Metallchelate. Insbesondere geeignet sind AlQ₃ (Tris(8-hydroxychinolato)aluminium), BeBq₂, 1,3,4-Oxidazolderivate (OXDs) wie PBD und 1,2,4-Triazole (TAZs). Desweiteren sind Bis(benzimidazolyl)-Derivate von Peryldendicarboximid (PD), Naphthalindicarboximid (ND) und Thiopyransulfone (TPS) geeignet. Bevorzugt eingesetzte elektronentransportierende Materialien sind z. B. in EP-A 1 138 745 offenbart.

Um die Stabilität des erfindungsgemäßen OLEDs gegenüber Temperatur, Feuchtigkeit und anderen Einflüssen zu erhöhen, kann das OLED durch eine Schutzschicht auf der Oberfläche des OLED geschützt sein, wobei diese Schutzschicht z. B. aus einem Harz oder Silikonöl aufgebaut ist.

Als leitendes Material, das für die Anode des erfindungsgemäßen OLEDs geeignet ist, wird bevorzugt Material eingesetzt, das eine Arbeitsfunktion von ≥ 4 eV aufweist. Geeignete Materialien für die Anode sind z. B. Kohlenstoff, Vanadium, Eisen, Kobalt, Nickel, Wolfram, Gold, Platin, Palladium und Legierungen dieser Materialien, Metalloxide wie sie für ITO-Substrate (ITO = Indium-Zinn-Oxid) und NESA-Substrate verwendet werden, wie Zinnoxide und Indiumoxide und organische leitende Polymere wie Polythiophen und Polypyrrol.

Als leitendes Material für die Kathode ist Material geeignet, das eine Arbeitsfunktion von < 4 eV aufweist. Für die Kathode geeignete Materialien sind beispielsweise Magnesium, Kalzium, Zinn, Blei, Titan, Yttrium, Lithium, Ruthenium, Mangan, Aluminium und Legierungen dieser Materialien.

Die Anode und die Kathode können gegebenenfalls eine Multischichtstruktur aus zwei oder mehreren Schichten aufweisen.

Die OLEDs der vorliegenden Erfindung weisen bevorzugt zusätzlich eine Schicht eines Chalkogenids, eines Metallhalogenids oder eines Metalloxids auf der Oberfläche mindestens eines Elektrodenpaares auf. Besonders bevorzugt ist eine Schicht eines Chalkogenids (inklusive eines Oxids) eines Metalls, z. B. Silizium oder Aluminium, auf die Oberfläche der Anode auf der Seite aufgebracht, die in die Richtung der Lichtemittierenden Schicht zeigt. Auf die Oberfläche der Kathode, die in die Richtung der Lichtemittierenden Schicht zeigt, wird bevorzugt eine Schicht eines Metallhalogenids oder eines Metalloxids aufgebracht. Aufgrund der beiden oben genannten Schichten kann die Stabilität der OLEDs verbessert werden. Bevorzugte Materialien für die genannten Schichten sind z. B. in EP-A 1 138 745 genannt.

Weitere bevorzugt Ausführungsformen der einzelnen Schichten der OLEDs sind ebenfalls in EP-A 1 138 745 aufgeführt.

Im Allgemeinen ist mindestens eine Seite der erfindungsgemäßen OLED in dem Wellenlängenbereich, in dem Licht emittiert werden soll, transparent, um eine effiziente Lichtemission zu ermöglichen. Die transparente Elektrode wird im Allgemeinen durch vapor deposition oder Sputtering aufgebracht. Bevorzugt weist die Elektrode auf der lichtemittierenden Seite der OLED eine Durchlässigkeit für Licht von ≥ 10% auf. Geeignete Materialien sind dem Fachmann bekannt. Beispielsweise können Glas-Substrate oder transparente Polymerfilme eingesetzt werden.

Die Herstellung der erfindungsgemäßen OLEDs ist dem Fachmann bekannt. Es ist möglich, dass jede Schicht der OLED durch ein trockenes Verfahren zur Filmbildung wie vapor deposition, Sputtering, Plasma plating oder Ion plating oder ein nasses Verfahren der Filmbildung wie Spincoating, Tauchen oder Flowcoating hergestellt wird. Die Dicke der einzelnen Schichten ist nicht begrenzt und übliche Dicken sind dem Fachmann bekannt. Geeignete Dicken der Schichten liegen im Allgemeinen im Bereich von 5 nm bis 10 µm Bevorzugt sind Dicken von 10 nm bis 0,2 µm. Die Durchführung von trockenen Verfahren oder nassen Verfahren zur Filmbildung sind dem Fachmann bekannt.

Ein weiterer Gegenstand der vorliegenden Erfindung ist somit eine OLED enthaltend eine lichtemittierende Schicht, die ein oder mehrere Fluoranthenderivate der allgemeinen Formel (I) als Emittermoleküle enthält. Bevorzugte Verbindungen der allgemeinen Formel (I) sind bereits vorstehend genannt.

Die erfindungsgemäße OLED kann in zahlreichen Vorrichtungen eingesetzt werden. Somit ist ein weiterer Gegenstand der vorliegenden Erfindung eine Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen.

Die nachfolgenden Beispiele erläutern die Erfindung zusätzlich.

### Beispiele

Die Nomenklatur der Fluoranthene im Sinne der vorliegenden Erfindung erfolgt nach dem untenstehenden Schema:

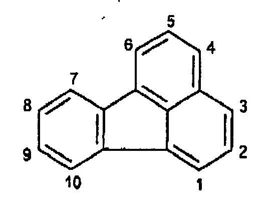

### Beispiel 1 (Vergleich)

### 3,8-Di-(2-thienyl)fluoranthen:

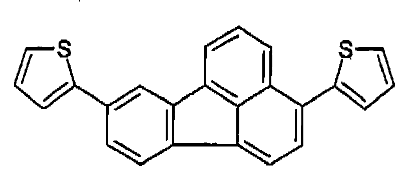

0,1 g 3,8-Dibromfluoranthen, hergestellt durch zweifache Bromierung von Fluoranthen in Dimethylformamid (DMF) mit Bromsuccinimid (NBS), wurden unter Schutzgas in 25 ml getrocknetem Toluol gelöst und mehrfach entgast. 4,9 g Kaliumcarbonat, gelöst in 18 ml Ethanol/Wasser (1:1 Volumenanteile) und 0,1 g Thiopben-2-bornsäure wurden zugegeben und erneut entgast. Anschließend wurden unter Schutzgas 0,04g Tetrakis(triphenylphosphin)palladium(0) (Pd(PPh₃)₄) zugegeben und 16 Stunden bei 80°C gerührt. Das Reaktionsgemisch wurde auf Methanol gegossen, filtriert und das Filtrat nach Trocknung mit MgSO₄ eingeengt und an Kieselgel (Merck Kieselgel 60) chromatographisch gereinigt. Unpolare Verunreinigungen wurden mit Cyclohexan eluiert, das Produkt anschließend mit Ethylacetat/Cyclohexan (1:50 Volumenanteile) eluiert. Ausbeute: 40% als gelber Feststoff. Die Struktur wurde durch FD-Massenspektrometrie bestätigt. In Lösung (Toluol) zeigt die Verbindung eine mit dem Auge sichtbare gelborangefarbene Fluoreszenz.

### Beispiel 2

### Bis-3,8-(3,5-difluorophenyl)fluoranthen:

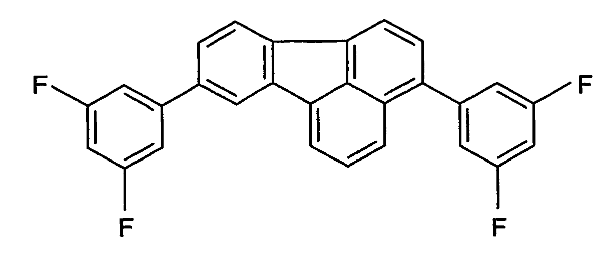

0,5 g 3,8-Dibromfluoranthen, hergestellt durch zweifache Bromierung von Fluoranthen in Chloroform mit elementarem Brom, wurden unter Schutzgas in 22 ml getrocknetem Toluol gelöst und mehrfach entgast. 9,7 g Kaliumcarbonat, gelöst in 28 ml Ethanol/Wasser (1:1 Volumenanteile) und 0,48 g 3,5-Difluorbenzolboronsäure wurden zugegeben und erneut entgast. Anschließend wurden unter Schutzgas 0,04 g Tetrakis(triphenylphosphin)palladium(0) (Pd(PPh₃)₄) zugegeben und 16 Stunden bei 80°C gerührt. Das Reaktionsgemisch wurde auf Methanol gegossen, filtriert und das Filtrat nach Trocknung mit MgSO₄ eingeengt und an Kieselgel (Merck Kieselgel 60) zweimal chromatographisch gereinigt. Unpolare Verunreinigungen wurden mit Cyclohexan eluiert, das Produkt anschließend mit Ethylacetat/Cyclohexan (1:50 Volumenanteile) eluiert. Ausbeute: 16% als gelber Feststoff. Die Struktur wurde massenspektrometrisch (Direktverdampfung) bestätigt. In Lösung (Toluol) zeigt die Verbindung bei einer Wellenlänge von λ_{max,em} (Toluol) = 468 nm Fluoreszenz bei einer Quantenausbeute (Toluol) von 43%.

### Beispiel 3

### 3,8-Di-(4-nitrouhenyl)fluoranthen:

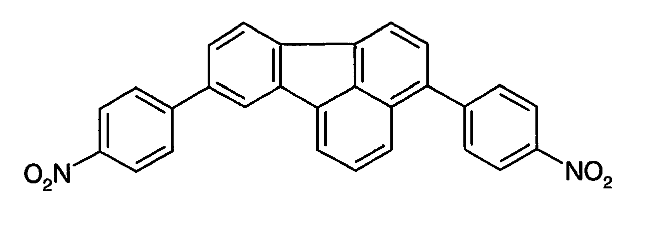

1 g 3,8-Dibromfluoranthen, hergestellt durch zweifache Bromierung von Fluoranthen in Chloroform mit elementarem Brom, wurden unter Schutzgas in 43 ml getrocknetem Toluol gelöst und mehrfach entgast. 19,4 g Kaliumcarbonat, gelöst in 47 ml Ethanol/Wasser (1:1 Volumenanteile) und 1 g 4-Nitrobenzolboronsäure wurden zugegeben und erneut entgast. Anschließend wurden unter Schutzgas 0,32 g Tetrakis(triphenylphosphin)palladium(0) (Pd(PPh₃)₄) zugegeben und 16 Stunden bei 80°C gerührt. Das Reaktionsgemisch wurde auf Methanol gegossen, filtriert und das Filtrat nach Trocknung mit MgSO₄ eingeengt und an Kieselgel (Merck Kieselgel 60) chromatographisch gereinigt. Unpolare Verunreinigungen wurden mit Cyclohexan eluiert, das Produkt anschließend mit Ethylacetat/Cyclohexan (1:2 Volumenanteile) eluiert. Ausbeute: 14% als gelber Feststoff. Die Struktur wurde massenspektrometrisch (Direktverdampfung) bestätigt. In Lösung (Toluol) zeigt die Verbindung bei einer Wellenlänge von λ_{max,em} (Toluol) = 471 nm Fluoreszenz bei einer Quantenausbeute (Toluol) von 3%.

### Beispiel 4

### 3,8-Bis-(biphen-4-yl)fluoranthen:

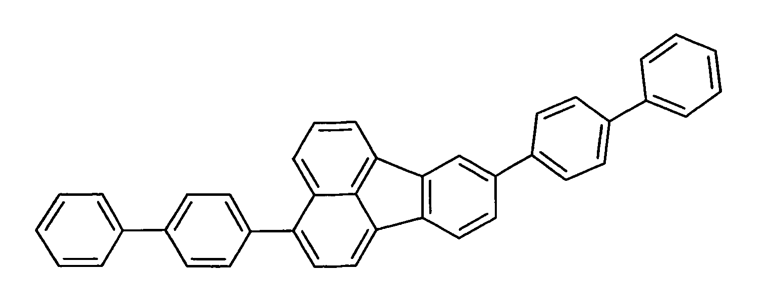

1 g 3,8-Dibromfluoranthen, hergestellt durch zweifache Bromierung von Fluoranthen in Chloroform mit elementarem Brom, wurden unter Schutzgas in 100 ml getrocknetem Toluol gelöst und mehrfach entgast. 19,4 g Kaliumcarbonat, gelöst in 47 ml Ethanol/Wasser (1:1 Volumenanteile) und 1 g 4-Biphenylboronsäure wurden zugegeben und erneut entgast. Anschließend wurden unter Schutzgas 0,32 g Tetrakis(triphenylphosphin)palladium(0) (Pd(PPh₃)₄) zugegeben und 16 Stunden bei 80°C gerührt. Das Reaktionsgemisch wurde filtriert und getrocknet. Ausbeute: 14% als gelblicher Feststoff. λ_{max,em} (Toluol) = 479 nm, Quantenausbeute (Toluol): 74%.

## Patentansprüche

1. Verwendung von Fluoranthenderivaten der allgemeinen Formel (I) 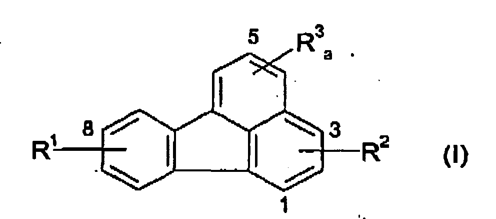 worin a = 0 ist und R¹ und R² unabhängig voneinander die folgenden Bedeutungen aufweisen:
R¹, R² eine mit linearen, verzweigten oder zyklischen C₁- bis C₂₀-Alkylgruppen, die wiederum mit Halogen-, Nitro-, Ether- oder Carboxygruppen substituiert oder unsubstituiert sein können und/oder worin ein oder mehrere Kohlenstoffatome der Alkylgruppen durch Si, P, O oder S ersetzt sein können, mit Alkoxygruppen oder mit C₆- bis C₁₄-Arylgruppen substituierte oder unsubstituierte C₆-Arylgruppe, und
wobei die Fluoranthenderivate Substituenten aufweisen, die über eine C-C-Einfachbindung mit dem Fluoranthengerüst verknüpft sind, als Emittermolekül in organischen Licht-emittierenden Dioden (OLEDs).

2. Verwendung von Fluoranthenderivaten nach Anspruch 1, **dadurch gekennzeichnet, dass** das Fluorantbenderivat der allgemeinen Formel (I) in der 3-Postion mit R² und in der 8-Position mit R¹ substituiert ist.

3. Verwendung von Fluoranthenderivaten nach Anspruch 1 oder 2, **dadurch gekennzeichnet, dass** R¹ und R² unabhängig voneinander eine mit linearen oder verzweigten unsubstituierten C₁- bis C₈-Alkylgruppen, Halogen- oder Nitrogruppen substituierte oder unsubstituierte Phenylgruppe bedeuten.

4. Verwendung von Fluoranthenderivaten nach Anspruch 3, **dadurch gekennzeichnet, dass** die Phenylgruppe keinen, einen oder zwei Substituenten trägt.

5. Verfahren zur Herstellung von Fluoranthenderivaten der allgemeinen Formel (I) 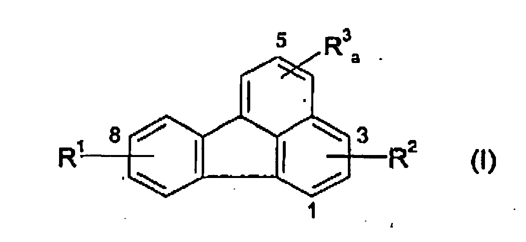 worin R¹, R², R³ und a unabhängig voneinander die folgenden Bedeutungen aufweisen:
R¹, R² und R³ Wasserstoff, eine lineare, verzweigte oder zyklische mit Halogen-, Nitro-, Ether- oder Carboxygruppen substituierte oder unsubstituierte C₁- bis C₂₀-Alkylgruppe, wobei ein oder mehrere nicht benachbarte Kohlenstoffatome der Alkylgruppe, das/die nicht unmittelbar an das Fluoranthengerüst gebunden ist/sind, durch Si, P, O oder S ersetzt sein können,
eine mit linearen, verzweigten oder zyklischen C₁- bis C₂₀-Alkylgruppen, die wiederum mit Halogen-, Nitro-, Ether- oder Carboxygruppen substituiert oder unsubstituiert sein könne und/oder worin ein oder mehrere Kohlenstoffatome der Alkylgruppen durch Si, P, O oder S ersetzt sein können, mit Alkoxygruppen oder mit C₆- bis C₁₄-Arylgruppen substituierte oder unsubstituierte C₆- bis C₃₀-Arylgruppe oder eine mit den bezüglich der Arylgruppe genannten Substituenten substituierte oder unsubstituierte C₄- bis C₁₄-Heteroarylgruppe, enthaltend mindestens ein N- oder S-Atom,
eine Gruppe der Formel (E)- oder (Z)-CH=CH-C₆R⁴₅, worin R⁴ H oder CH₃ ist, eine Gruppe der Formel 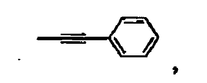 oder eine Acryloyl- oder Methacryloylgruppe, ein Vinyletherrest, und eine Gruppe der Formel 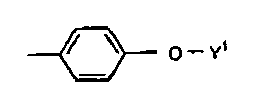 worin Y¹ -CH=CH₂, (E)- oder (Z)-CH=CH-C₆H₅, Acryloyl, Methacryloyl, Methylstyryl, -O-CH=CH₂ oder Glycidyl bedeutet,
einen annellierten Aromaten wie Naphthalin, Anthracen, Pyren, Phenanthren und Perylen, der mit einem oder mehreren Halogen-, Nitro-, Ether- oder Carboxylrest substituiert sein kann, und
a eine ganze Zahl von 0 bis 3,
mit der Maßgabe, dass mindestens einer der Reste R¹ oder R² nicht Wasserstoff ist, wobei die Fluoranthenderivaie Substituenten aufweisen, die über eine C-C-Einfachbindung mit dem Fluoranthengerüst verknüpft sind,
durch Umsetzung von an den Positionen, an denen in dem gewünschten Fluoranthenderivat der Formel (I) die Reste R¹, R² und gegebenenfalls R³ mit dem Fluoranthengerüst verknüpft sind, halogenierten Fluoranthenderivaten mit den den gewünschten Resten R¹, R² und gegebenenfalls R³ entsprechenden Boronsäuren unter Pd(0)-Katalyse in Gegenwart einer Base
oder
durch Umsetzung von an den Positionen, an denen in dem gewünschten Fluoranthenderivat der Formel (I) die Reste R¹, R² und gegebenenfalls R³ mit dem Fluoranthengerüst verknüpft sind, halogenierten Fluoranthenderivaten mit den den gewünschten Resten R¹, R² und gegebenenfalls R³ entsprechenden Brom-Verbindungen unter Ni(0)-Katalyse.

6. Lichtemittierende Schicht enthaltend ein oder mehrere Fluoranthenderivate der allgemeinen Fonnel (I), wie in einem der Ansprüche 1 bis 4 definiert, als Emittermoleküle.

7. OLED enthaltend eine Lichtemittierende Schicht gemäß Anspruch 6.

8. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen, enthaltend eine OLED gemäß Anspruch 7.

9. Fluoranthenderivate der allgemeinen Formel (I) 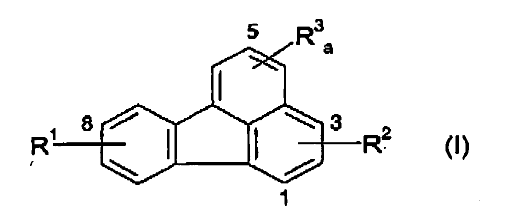
worin a = 0 ist und R¹ und R² unabhängig voneinander die folgenden Bedeutungen aufweisen:
R¹, R² eine mit linearen, verzweigten oder zyklischen C₁- bis C₂₀-Alkylgruppen, die wiederum mit Halogen-, Nitro-, Ether- oder Carboxygruppen substituiert oder unsubstituiert sein können und/oder worin ein oder mehrere Kohlenstoffatome der Alkylgruppen durch Si, P, O oder S ersetzt sein können, mit Alkoxygruppen oder mit C₆- bis C₁₄-Arylgruppen substituierte oder unsubstituierte C₆-Arylgruppe, wobei
das Fluoranthenderivat der allgemeinen Formel (I) in der 3-Postion mit R² und in der 8-Position mit R¹ substituiert ist.

10. Fluoranthenderivate nach Anspruch 9, **dadurch gekennzeichnet, dass** R¹ und R² unabhängig voneinander eine mit linearen oder verzweigten unsubstituierten C₁- bis C₆-Alkylgruppen, Halogen- oder Nitrogruppen substituierte oder unsubstituierte Phenylgruppe bedeuten.

11. Fluoranthenderivate nach Anspruch 10, **dadurch gekennzeichnet, dass** die Phenylgruppe keinen, einen oder zwei Substituenten trägt.

## Claims

1. The use of fluoranthene derivatives of the general formula (I) 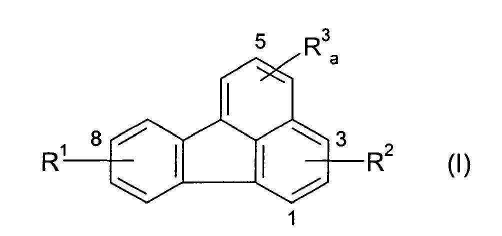 in which a = 0 and R¹ and R² are each independently defined as follows:
R¹, R² are each a C₆-aryl group which may be unsubstituted or substituted by linear, branched or cyclic C₁- to C₂₀-alkyl groups which may in turn be unsubstituted or substituted by halogen, nitro, ether or carboxyl groups and/or in which one or more carbon atoms of the alkyl groups may be replaced by Si, P, O or S, by alkoxy groups or by C₆- to C₁₄-aryl groups, and
where the fluoranthene derivatives have substituents which are bonded to the fluoranthene skeleton via a C-C single bond, as emitter molecules in organic light-emitting diodes (OLEDs).

2. The use of fluoranthene derivatives according to claim 1, wherein the fluoranthene derivative of the general formula (I) is substituted by R² in the 3-position and by R¹ in the 8-position.

3. The use of fluoranthene derivatives according to claim 1 or 2, wherein R¹ and R² are each independently a phenyl group which is unsubstituted or substituted by linear or branched, unsubstituted C₁- to C₈-alkyl groups, halogen or nitro groups.

4. The use of fluoranthene derivatives according to claim 3, wherein the phenyl group bears no, one or two substituents.

5. A process for preparing fluoranthene derivatives of the general formula (I) 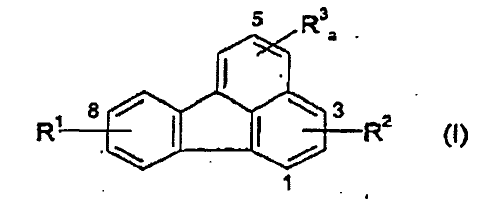 in which R¹, R², R³ and a are each independently defined as follows:
R¹, R² and R³ are each hydrogen, a linear, branched or cyclic, halogen-, nitro-, ether- or carboxyl-substituted or unsubstituted C₁- to C₂₀-alkyl group, in which one or more nonadjacent carbon atoms of the alkyl group which is/are not bonded directly to the fluoranthene skeleton may be replaced by Si, P, O or S,
an alkoxy- or C₆- to C₁₄-aryl-substituted or unsubstituted C₆- to C₃₀-aryl group which may be unsubstituted or substituted by linear, branched or cyclic C₁- to C₂₀-alkyl groups which may be unsubstituted or may in turn be substituted by halogen, nitro, ether or carboxyl groups and/or in which one or more carbon atoms of the alkyl groups may be replaced by Si, P, O or S, or a C₄- to C₁₄-heteroaryl group which is unsubstituted or substituted by the substituents specified with regard to the aryl group and comprises at least one nitrogen or sulfur atom,
a group of the formula (E)- or (Z)-CH=CH-C₆R⁴₅ in which R⁴ is H or CH₃, a group of the formula 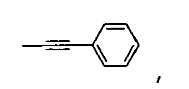
or an acryloyl or methacryloyl group, a vinyl ether radical and a group of the formula 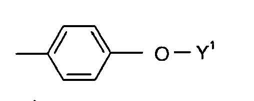
in which Y¹ is -CH=CH₂, (E)- or (Z)-CH=CH-C₆H₅, acryloyl, methacryloyl, methylstyryl, -O-CH=CH₂ or glycidyl,
a fused aromatic such as naphthalene, anthracene, pyrene, phenanthrene and perylene, which may be substituted by one or more halogen, nitro, ether or carboxyl radicals, and
a is an integer from 0 to 3,
with the proviso that at least one of the R¹ or R² radicals is not hydrogen,
where the fluoranthene derivatives have substituents which are bonded to the fluoranthene skeleton via a C-C single bond,
by reacting fluoranthene derivatives halogenated at the positions at which the R¹, R² and, if appropriate, R³ radicals are bonded to the fluoranthene skeleton in the desired fluoranthene derivative of the formula (I) with the boronic acids corresponding to the desired R¹, R² and, if appropriate, R³ radicals under Pd(0) catalysis in the presence of a base
or
by reacting fluoranthene derivatives halogenated at the positions at which the R¹, R² and, if appropriate, R³ radicals are bonded to the fluoranthene skeleton in the desired fluoranthene derivative of the formula (I) with the bromine compounds corresponding to the desired R¹, R² and, if appropriate, R³ radicals under Ni(0) catalysis.

6. A light-emitting layer comprising one or more fluoranthene derivatives of the general formula (I) as defined in any of claims 1 to 4 as emitter molecules.

7. An OLED comprising a light-emitting layer according to claim 6.

8. A device selected from the group consisting of stationary visual display units such as visual display units of computers, televisions, visual display units in printers, kitchen appliances, and advertising panels, illuminations, information panels and mobile visual display units such as visual display units in cellphones, laptops, vehicles, and destination displays on buses and trains, comprising an OLED according to claim 7.

9. A fluoranthene derivative of the general formula (I) 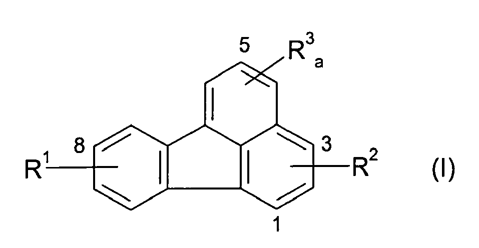 in which a = 0 and R¹ and R² are each independently defined as follows:
R¹, R² are each a C₆-aryl group which may be unsubstituted or substituted by linear, branched or cyclic C₁- to C₂₀-alkyl groups which may in turn be unsubstituted or substituted by halogen, nitro, ether or carboxyl groups and/or in which one or more carbon atoms of the alkyl groups may be replaced by Si, P, O or S, by alkoxy groups or by C₆- to C₁₄-aryl groups, where
the fluoranthene derivative of the general formula (I) is substituted by R² in the 3-position and by R¹ in the 8-position.

10. The fluoranthene derivative according to claim 9, wherein R¹ and R² are each independently a phenyl group which is unsubstituted or substituted by linear or branched, unsubstituted C₁- to C₈-alkyl groups, halogen or nitro groups.

11. The fluoranthene derivative according to claim 10, wherein the phenyl group bears no, one or two substituents.

## Revendications

1. Utilisation de dérivés de fluoranthène de formule générale (I) 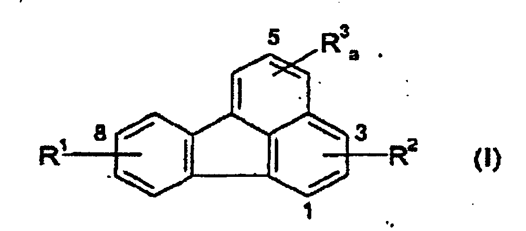 dans laquelle a = 0 et R¹ et R² ont, indépendamment l'un de l'autre, les significations suivantes :
R¹, R² représentent un groupe aryle en C₆ non substitué ou substitué par des groupes alkyle en C₁-C₂₀ linéaires, ramifiés ou cycliques, qui peuvent à leur tour être non substitués ou substitués par des groupes halogéno, nitro, éther ou carboxy et/ou dans lesquels un ou plusieurs atomes de carbone des groupes alkyle peuvent être remplacés par Si, P, O ou S, ou substitué par des groupes alcoxy ou par des groupes aryle en C₆-C₁₄, et
les dérivés de fluoranthène comportant des substituants qui sont liés par une simple liaison C-C au squelette fluoranthène, en tant que molécule émettrice dans des diodes électroluminescentes organiques (OLED).

2. Utilisation de dérivés de fluoranthène selon la revendication 1, **caractérisée en ce que** le dérivé de fluoranthène de formule générale (I) est substitué en la position 3 par R² et en la position 8 par R¹.

3. Utilisation de dérivés de fluoranthène selon la revendication 1 ou 2, **caractérisée en ce que** R¹ et R² représentent, indépendamment l'un de l'autre, un groupe phényle non substitué ou substitué par des groupes alkyle en C₁-C₈ linéaires ou ramifiés, non substitués, des groupes halogéno ou nitro.

4. Utilisation de dérivés de fluoranthène selon la revendication 3, **caractérisée en ce que** le groupe phényle est non substitué ou porte un ou deux substituants.

5. Procédé pour la préparation de dérivés de fluoranthène de formule générale (I) 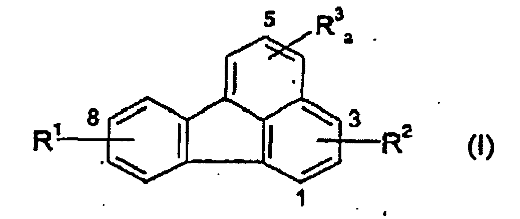 dans laquelle R¹, R², R³ et a ont, indépendamment les uns des autres, les significations suivantes :
R¹, R² et R³ représentent un atome d'hydrogène, un groupe alkyle en C₁-C₂₀ linéaire, ramifié ou cyclique, non substitué ou substitué par des groupes halogéno, nitro, éther ou carboxy, un ou plusieurs atomes de carbone non contigus du groupe alkyle, qui n'est/ne sont pas lié(s) directement au squelette fluoranthène, peut/peuvent être remplacé(s) par Si, P, O ou S,
un groupe aryle en C₆-C₃₀ non substitué ou substitué par des groupes alkyle en C₁-C₂₀ linéaires, ramifiés ou cycliques, qui peuvent à leur tour être non substitués ou substitués par des groupes halogéno, nitro, éther ou carboxy et/ou dans lesquels un ou plusieurs atomes de carbone des groupes alkyle peuvent être remplacés par Si, P, O ou S, ou substitué par des groupes alcoxy ou par des groupes aryle en C₆-C₁₄,
ou un groupe hétéroaryle en C₄-C₁₄ contenant au moins un atome d'azote ou de soufre, non substitué ou substitué par les substituants nommés à propos du groupe aryle,
un groupe de formule (E)- ou (Z)-CH=CH-C₆R⁴₅, R⁴ étant H ou CH₃, un groupe de formule 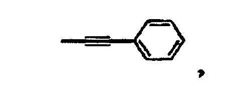
ou un groupe acryloyle ou méthacryloyle, un radical éther vinylique, et
un groupe de formule 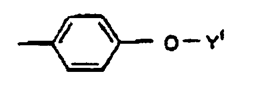
dans laquelle Y¹ représente -CH=CH₂, (E)- ou (Z)-CH=CH-C₆H_{5,} un groupe acryloyle, méthacryloyle, méthylstyryle, -O-CH=CH₂ ou glycidyle,
un composé aromatique soudé tel que naphtalène, anthracène, pyrène, phénanthrène et pérylène, qui peut être substitué par un ou plusieurs radicaux halogéno-, nitro, éther ou carboxy, et
a est un nombre entier allant de 0 à 3,
étant entendu qu'au moins l'un des radicaux R¹ ou R² n'est pas un atome d'hydrogène,
les dérivés de fluoranthène comportant des substituants qui sont liés par une simple liaison C-C au squelette fluoranthène,
par mise en réaction de dérivés de fluoranthène halogénés en les positions en lesquelles les radicaux R¹, R² et éventuellement R³, dans le dérivé de fluoranthène de formule (I) recherché, sont liés au squelette fluoranthène, avec les acides boroniques correspondant aux radicaux R¹, R² et éventuellement R³ désirés, avec catalyse par Pd(0) en présence d'une base
ou
par mise en réaction de dérivés de fluoranthène halogénés en les positions en lesquelles les radicaux R¹, R² et éventuellement R³, dans le dérivé de fluoranthène de formule (I) recherché, sont liés au squelette fluoranthène, avec les composés bromés correspondant aux radicaux R¹, R² et éventuellement R³ désirés, avec catalyse par Ni(0).

6. Couche électroluminescente contenant un ou plusieurs dérivés de fluoranthène de formule générale (I), tels que définis dans l'une quelconque des revendications 1 à 4, en tant que molécules émettrices.

7. OLED contenant une couche électroluminescente selon la revendication 6.

8. Dispositif choisi dans le groupe constitué par des écrans fixes tels que des écrans d'ordinateurs, des téléviseurs, des écrans dans des imprimantes, des appareils de cuisine ainsi que des tableaux publicitaires, des éclairages, des tableaux indicateurs et des écrans mobiles tels que des écrans dans des téléphones portables, des ordinateurs portables, des véhicules ainsi que des afficheurs de destination dans des autobus et des trains, contenant une OLED selon la revendication 7.

9. Dérivés de fluoranthène de formule générale (I) 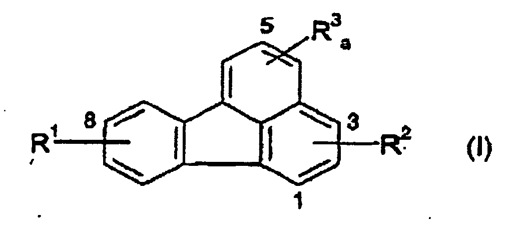 dans laquelle a = 0 et R¹ et R² ont, indépendamment l'un de l'autre, les significations suivantes :
R¹, R² représentent un groupe aryle en C₆ non substitué ou substitué par des groupes alkyle en C₁-C₂₀ linéaires, ramifiés ou cycliques, qui peuvent à leur tour être non substitués ou substitués par des groupes halogéno, nitro, éther ou carboxy et/ou dans lesquels un ou plusieurs atomes de carbone des groupes alkyle peuvent être remplacés par Si, P, O ou S, ou substitué par des groupes alcoxy ou par des groupes aryle en C₆-C₁₄,
le dérivé de fluoranthène de formule générale (I) étant substitué en la position 3 par R² et en la position 8 par R¹.

10. Dérivés de fluoranthène selon la revendication 9, **caractérisés en ce que** R¹ et R² représentent, indépendamment l'un de l'autre, un groupe phényle non substitué ou substitué par des groupes alkyle en C₁-C₈ linéaires ou ramifiés, non substitués, des groupes halogéno ou nitro.

11. Dérivés de fluoranthène selon la revendication 10, **caractérisés en ce que** le groupe phényle est non substitué ou porte un ou deux substituants.
